# EUROPEAN PATENT SPECIFICATION

(11) **EP 1 924 598 B1**
(45) Date of publication and mention of the grant of the patent: **17.11.2010**
(21) Application number: 06798077.1
(22) Date of filing: 12.09.2006
(51) Int. Cl.: C07K 14/435, C12N 15/09

(54) **ESTERASE GENE AND USE THEREOF**
ESTERASEGEN UND ANWENDUNG DAVON
GENE ESTERASE ET UTILISATION ASSOCIEE

(30) Priority: 13.09.2005 JP 2005265918; 23.02.2006 JP 2006047561
(43) Date of publication of application: 28.05.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 618-8503 (JP); KODAMA, Yukiko, Mishima-gun, Osaka 618-8503 (JP); SHIMONAGA, Tomoko, Mishima-gun, Osaka 618-8503 (JP)
(74) Representative: Vossius & Partner
(86) International application number: PCT/JP2006/318466
(87) International publication number: WO 2007/032523

(56) References cited:
- WO-A2-2004/064537
- JP-A- 9 234 077

## Description

### TECHNICAL FIELD

The present invention relates to a esterase gene and to uses of the gene. The invention relates in particular to a brewer's yeast which produces alcoholic beverages with excellent aroma and flavor, alcoholic beverages produced using such a yeast, and a method of producing such alcoholic beverages. More specifically, the invention relates to LAH1 gene which codes for the esterase Iahlp in brewer's yeast, particularly to a yeast whose capability of producing ester, which contribute to aroma and flavor of a product, is controlled by regulating the level of expression of the nonScIAH1 gene characteristic to beer yeast and to a method of producing alcoholic beverages using such a yeast.

### BACKGROUND ART

Esters are an important aromatic component of alcoholic beverages. In the case of rice wine, wine and whisky, an increase in the ester content is known to give the beverage a florid aroma as well as cause it to be evaluated highly for its flavor. On the other hand, although esters are an important aromatic component of beer as well, an excess amount of esters is disliked due to the resulting ester smell. Thus, it is important to suitably control the amount of ester formed according to the type of alcoholic beverage.

Yeast producing high levels of esters have been developed in the past for the purpose of increasing the ester content of alcoholic beverages. Examples of previously reported methods for effective isolation of yeast producing large amounts of esters include a method in which yeast is subjected (or not subjected) to mutagenic treatment to obtain a strain which produces large amounts of caproic acid and is resistant to drugs that inhibit fatty acid synthases such as cerulenin, as well as a strain which is resistant to leucine analogs such as 5,5,5-trifluoro-DL-leucine and produces large amounts of isoamyl alcohol and isoamyl acetate (Japanese Patent Application Laid-open No. 2002-253211), and a method in which a strain is acquired which is grown in medium containing a steroid having a pregnane backbone hydroxylated at position 3 (Japanese Patent Application Laid-open No. 2002-191355).

On the other hand, examples of previously reported methods involving the development of yeast utilizing genetic engineering techniques include expressing high levels of the alcohol acetyl transferase gene ATF1 of Saccharomyces cerevisiae in brewing yeast (Japanese Patent Application Laid-open No. H06-062849), inhibiting the expression of ATF1 (Japanese Patent Application Laid-open No. H06-253826), and increasing the amount of ester by destroying esterase gene EST2 in brewing yeast (Japanese Patent Application Laid-open No. H09-234077).

### DISCLOSURE OF INVENTION

As stated above, although a mutant strain is acquired for increasing the ester content of a product, there are cases in which unexpected delays in fermentation or increases in undesirable aromatic and flavor components are observed as a result thereof, thus creating problems in the development of yeast for practical application. Consequently, there is need for a method for breeding yeast capable of producing a desired amount of esters without impairing the fermentation rate or product quality.

As a result of conducting extensive studies to solve the above-mentioned problems, the inventor of the present invention succeeded in identifying and isolating a gene from brewer's yeast which encodes an esterase that demonstrates more advantageous effects than known proteins. In addition, the inventors of the present invention also confirmed that the amount of ester formed decreases by producing a transformed yeast by inserting and expressing the resulting gene in yeast, and that the amount of ester formed increases by producing a transformed yeast in which expression of the resulting gene is suppressed, thereby leading to completion of the present invention.

Namely, the present invention relates to a novel esterase gene characteristically present in brewer's yeast, a protein encoded by said gene, a transformed yeast in which the expression of said gene is regulated, and a method for controlling the amount of ester formed in a product by using yeast in which expression of said gene has been regulated. More specifically, the present invention provides the polynucleotide indicated below, a vector containing said polynucleotide, a transformed yeast in which said vector has been inserted, and a method for producing an alcoholic beverage using said transformed yeast.
(1) A polynucleotide selected from the group consisting of
   (a) a polynucleotide comprising a polynucleotide consisting of the nucleotide sequence of SEQ ID NO:1;
   (b) a polynucleotide comprising a polynucleotide encoding a protein consisting of the amino acid sequence of SEQ ID NO:2;
   (c) a polynucleotide comprising a polynucleotide encoding a protein consisting of the amino acid sequence of SEQ ID NO:2 with one to 40 amino acids thereof being deleted, substituted, inserted and/or added, and having a esterase activity;
   (d) a polynucleotide comprising a polynucleotide encoding a protein having an amino acid sequence having 80% or higher identity with the amino acid sequence of SEQ ID NO:2, and having a esterase activity;
(2) The polynucleotide of (1) above selected from the group consisting of:
   (g) a polynucleotide encoding a protein consisting of the amino acid sequence of SEQ ID NO: 2, or encoding an amino acid sequence of SEQ ID NO: 2 wherein 1 to 10 amino acids thereof is deleted; substituted, inserted, and/or added, and wherein said protein has a esterase activity;
   (h) a polynucleotide encoding a protein having 90% or higher identity with the amino acid sequence of SEQ ID NO: 2, and having a esterase activity; and
(3) The polynucleotide of (1) above comprising a polynucleotide consisting of SEQ ID NO: 1.
(4) The polynucleotide of (1) above comprising a polynucleotide encoding a protein consisting of SEQ ID NO: 2.
(5) The polynucleotide of any one of (1) to (4) above, wherein the polynucleotide is DNA.
(6) A polynucleotide selected from the group consisting of:
   (j) a polynucleotide encoding RNA of a nucleotide sequence complementary to a transcript of the polynucleotide (DNA) according to (5) above;
   (k) a polynucleotide encoding RNA that represses the expression of the polynucleotide (DNA) according to (5) above through RNAi effect;
   (l) a polynucleotide encoding RNA having an activity of specifically cleaving a transcript of the polynucleotide (DNA) according to (5) above; and
   (m) a polynucleotide encoding RNA that represses expression of the polynucleotide (DNA) according to (5) above through co-suppression effect.
(7) A protein encoded by the polynucleotide of any one of (1) to (5) above.
(8) A vector comprising the polynucleotide of any one of (1) to (5) above.
(8a) The vector of (8) above, which comprises the expression cassette comprising the following components:
   (x) a promoter that can be transcribed in a yeast cell;
   (y) any of the polynucleotides described in (1) to (5) above linked to the promoter in a sense direction; and
   (z) a signal that can function in a yeast with respect to transcription termination and polyadenylation of a RNA molecule.
(9) A vector comprising the polynucleotide of (6) above.
(10) A yeast, wherein the vector of (8) or (9) above is introduced.
(11) The yeast of (10) above, wherein ester-producing ability is decreased by introducing the vector of (8) above.
(12) A yeast, wherein an expression of the polynucleotide (DNA) of (5) above is repressed by introducing the vendor of (9) above, or by disrupting a gene related to the polynucleotide (DNA) of (5) above.
(13) The yeast of (11) above, wherein a ester-producing ability is decreased by increasing an expression level of the protein of (7) above.
(14) A method for producing an alcoholic beverage by using the yeast of any one of (10) to (13) above.
(15) The method for producing an alcoholic beverage of (14) above, wherein the brewed alcoholic beverage is a malt beverage.
(16) The method for producing an alcoholic beverage of (14) above, wherein the brewed alcoholic beverage is a wine.
(17) An alcoholic beverage, which is produced by the method of any one of (14) to (16) above.
(18) A method for assessing a test yeast for its ester-producing capability, comprising using a primer or a probe designed based on a nucleotide sequence of a esterase gene having the nucleotide sequence of SEQ ID NO: 1.
(18a) A method for selecting a yeast having a target ester-producing capability by using the method in (18) above.
(18b) A method for producing an alcoholic beverage (for example, beer) by using the yeast selected with the method in (18a) above.
(19) A method for assessing a test yeast for its ester-producing capability, comprising:
   culturing a test yeast; and measuring an expression level of a esterase gene having the nucleotide sequence of SEQ ID NO: 1.
(20) A method for selecting a yeast, comprising: culturing test yeasts; quantifying the protein of (7) above or measuring an expression level of a esterase gene having the nucleotide sequence of SEQ ID NO: 1; and selecting a test yeast having said protein amount or said gene expression level according to a target capability of producing ester.
(20a) A method for selecting a yeast, comprising: culturing test yeasts; measuring a ester-producing capability or a esterase activity; and selecting a test yeast having a target capability of producing ester or a target esterase activity.
(21) The method for selecting a yeast of (20) above, comprising: culturing a reference yeast and test yeasts; measuring an expression level of a esterase gene having the nucleotide sequence of SEQ ID NO: 1 in each yeast; and selecting a test yeast having the gene expressed higher or lower than that in the reference yeast.
(22) The method for selecting a yeast of (20) above comprising: culturing a reference yeast and test yeasts; quantifying the protein of (7) above in each yeast; and selecting a test yeast having said protein for a larger or smaller amount than that in the reference yeast. That is, the method for selecting a yeast of (20) above comprising: culturing plural yeasts; quantifying the protein of (7) above in each yeast; and selecting a test yeast having a large or small amount of the protein from them.
(23) A method for producing an alcoholic beverage comprising: conducting fermentation for producing an alcoholic beverage using the yeast according to any one of (10) to (13) or a yeast selected by the method according to any one of (20) to (22); and adjusting the production amount of ester.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test.
   The horizontal axis represents fermentation time while the vertical axis represents apparent extract concentration (w/w%).
Figure 3 shows the expression profile of nonScIAH1 gene in yeasts upon beer fermentation test. The horizontal axis represents fermentation time while the vertical axis represents the intensity of detected signal.
Figure 4 shows the cell growth with time upon fermentation test. The horizontal axis represents fermentation time while the vertical axis represents optical density at 660 nm (OD660). The symbol "IAH1" denotes a nonScIAH1 highly expressed strain.
Figure 5 shows the extract (sugar) consumption with time upon beer fermentation test.
   The horizontal axis represents fermentation time while the vertical axis represents apparent extract concentration (w/w%). The symbol "IAH1" denotes a nonScIAH1 highly expressed strain.

Figure 6 shows the cell growth with time upon fermentation test. The horizontal axis represents fermentation time while the vertical axis represents optical density at 660 nm (OD660). The symbol "iah1" denotes a nonScIAH1 disrupted strain.
Figure 7 shows the extract (sugar) consumption with time upon beer fermentation test.
   The horizontal axis represents fermentation time while the vertical axis represents apparent extract concentration (w/w%). The symbol "iah1" denotes a nonScIAH1 disrupted strain.

### BEST MODES FOR CARRYING OUT THE INVENTION

The present inventors conceived that it is possible to control ester in products by increasing or decreasing a esterase activity of the yeast. The present inventors have studied based on this conception and as a result, isolated and identified a nonScIAH1 gene encoding a esterase unique to lager brewing yeast based on the lager brewing yeast genome information mapped according to the method disclosed in Japanese Patent Application Laid-Open No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide of the nucleotide sequence of SEQ ID NO:1; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding a esterase gene derived from lager brewing yeast described above and may include other polynucleotides encoding proteins' having equivalent functions to said protein. Proteins with equivalent functions include, for example, (c) a protein of an amino acid sequence of SEQ ID NO: 2 with one or more amino acids thereof being deleted, substituted, inserted and/or added and having a esterase activity.

Such proteins include a protein consisting of an amino acid sequence of SEQ ID NO: 2 with, for example, 1 to 100, 1 to 90, 1 to 80, 1 to 70, 1 to 60, 1 to 50, 1 to 40, 1 to 39, 1 to 38, 1 to 37, 1 to 36, 1 to 35, 1 to 34, to 33, 1 to 32, 1 to 31, 1 to 30, 1 to 29, 1 to 28, 1 to 27, 1 to 26, 1 to 25, 1 to 24, 1 to 23, 1 to 22, 1 to 21, 1 to 20, 1 to 19, 1 1 to 18, 1 1 to 17, 1 to 16, 1 to 15, 1 to 14, 1 to 13, 1 to 12, 1 to 11, 1 to 10, 1 to 9, 1 to 8, 1 1 to 7, 1 to 6 (1 to several amino acids), 1 to 5, 1 to 4, 1 to 3, 1 to 2, or 1 amino acid residues thereof being deleted, substituted, inserted and/or added and having a esterase activity. In general, the number of deletions, substitutions, insertions, and/or additions is preferably smaller. In addition, such proteins include (d) a protein having an amino acid sequence with about 60% or higher, about 70% or higher, 71 % or higher, 72% or higher, 73% or higher, 74% or higher, 75% or higher, 76% or higher, 77% or higher, 78% or higher, 79% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having a esterase activity. In general, the percentage identity is preferably higher.

Esterase activity may be measured, for example, by a method described in Appl. Microbiol. Biotechnol. 53: 596-600, 2000.

Furthermore, the present invention also contemplates (e) a polynucleotide comprising a polynucleotide which hybridizes to a polynucleotide consisting of a nucleotide sequence complementary to the nucleotide sequence of SEQ ID NO: 1 under stringent conditions and which encodes a protein having a esterase activity; and (f) a polynucleotide comprising a polynucleotide which hybridizes to a polynucleotide complementary to a nucleotide sequence of encoding a protein of SEQ ID NO: 2 under stringent conditions, and which encodes a protein having a esterase activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or polynucleotide encoding the amino acid sequence of SEQ ID NO: 2 as a probe. The hybridization method may be a method descried, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primacy wash buffer containing 0.1% (w/v) SDS at 55°C, thereby detecting hybridized polynucleotide, such as DNA.

Other polynucleotides that can be hybridized include polynucleotides having about 60% or higher, about 70% or higher, 71% or higher, 72% or higher, 73% or higher, 74% or higher, 75% or higher, 76% or higher, 77% or higher, 78% or higher, 79% or higher, 80% or higher, 81 % or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (Proc. Natl. Acad Sci. USA, 87: 2264-2268, 1990; Proc. Natl. Acad Sci. USA, 90: 5873, 1993). Programs called BLASTN and BLASTX based on BLAST algorithm have been developed (Altschul SF et al., J. Mol. Biol. 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

The polynucleotide of the present invention includes (j) a polynucleotide encoding RNA having a nucleotide sequence complementary to a transcript of the polynucleotide (DNA) According to (5) above; (k) a polynucleotide encoding RNA that represses the expression of the polynucleotide (DNA) according to (5) above through RNAi effect; (I) a polynucleotide encoding RNA having an activity of specifically cleaving a transcript of the polynucleotide (DNA) according to (5) above; and (m) a polynucleotide encoding RNA that represses expression of the polynucleotide (DNA) according to (5) above through co-suppression effect. These polynucleotides may be incorporated into a vector, which can be introduced into a cell for transformation to repress the expression of the polynucleotides (DNA) of (a) to (i) above. Thus, these polynucleotides may suitably be used when repression of the expression of the above DNA is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347; 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucleotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof. Methods for producing or using such double-stranded RNA (dsRNA, siRNA or shRNA) are known from many publications (see, e.g., Japanese National Phase PCT Laid-open Patent Publication No. 2002-516062; US 2002/086356A; Nature Genetics, 24(2), 180-183, 2000 Feb.; Genesis, 26(4), 240-244, 2000 April; Nature, 407:6802, 319-20, 2002 Sep. 21; Genes .& Dev., Vol.16, (8), 948-958, 2002 Apr.15; Proc. Natl. Acad. Sci. USA., 99(8), 5515-5520, 2002 Apr. 16; Science, 296(5567), 550-553, 2002 Apr. 19; Proc NatL Acad. Sci. USA, 99:9, 6047-6052, 2002 Apr. 30; Nature Biotechnology, Vol.20 (5), 497-500, 2002 May; Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; NucL Acids. Res. 19: 6751, 1991; Protein Eng 3: 733,1990; Nucl. Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-suppression effect" refers to a nucleotide that inhibits functions of target DNA by "co-suppression".

The term "co-suppression" as used herein, refers to a phenomenon where when a gene having a sequence identical or similar to a target endogenous gene is transformed into a cell, the expressions of both the introduced foreign gene and the target endogenous gene are repressed Design of polynucleotides having a co-suppression effect can also be found in various publications (see, e.g., Smyth DR: Curr. BioL 7: R793, 1997, Martienssen R: Curr. BioL 6: 810, 1996).

### 2. Protein of the present invention

The present invention also provides proteins encoded by any of the polynucleotides (a) to (i) above. A preferred protein of the present invention comprises an amino acid sequence of SEQ ID NO:2 with one or several amino acids thereof being deleted, substituted, inserted and/or added, and has a esterase activity.

Such protein includes those having an amino acid sequence of SEQ ID NO: 2 with amino acid residues thereof of the number mentioned above being deleted, substituted, inserted and/or added and having a esterase activity. In addition, such protein includes those having homology as described above with the amino acid sequence of SEQ ID NO: 2 and having a esterase activity.

Such proteins may be obtained by employing site-directed mutation described, for example, in MOLECULAR CLONING 3rd Ed., CURRENT PROTOCOLS IN MOLECULAR BIOLOGY, Nuc. Acids. Res., 10: 6487 (1982), Proc. Natl. Acad. Sci: USA 79: 6409 (1982), Gene 34; 315 (1985), Nuc. Acids. Res., 13:4431 (1985), Proc. Natl. Acad. Sci. USA 82: 488 (1985).

Deletion, substitution, insertion and/or addition of one or more amino acid residues in an amino acid sequence of the protein of the invention means that one or more amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides described in (a) to (i) above or the polynucleotides described in (j) to (m) above. Generally, the vector of the present invention comprises an expression cassette including as components (x) a promoter that can transcribe in a yeast cell; (y) a polynucleotide described in any of (a) to (i) above that is linked to the promoter in sense or antisense direction; and (z) a signal that functions in the yeast with respect to transcription termination and polyadenylation of RNA molecule.

According to the present invention, in order to highly express the protein of the invention described above upon brewing alcoholic beverages (e.g., beer) described below, these polynucleotides are introduced in the sense direction to the promoter to promote expression of the polynucleotide (DNA) described in any of (a) to (i) above. Further, in order to repress the above protein of the invention upon brewing alcoholic beverages (e.g., beer) described below, these polynucleotides are introduced in the antisense direction to the promoter to repress the expression of the polynucleotide (DNA) described in any of (a) to (i) above. In order to repress the above protein of the invention, the polynucleotide may be introduced into vectors such that the polynucleotide of any of the (j) to (m) is to be expressed. According to the present invention, the target gene (DNA) may be disrupted to repress the expression of the DNA described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979) , Methods in Enzymology, 101,202(1983), Japanese Patent Application Laid-Open No.6-253826).

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et aL, Gene 60: 237, 1987) is known as a YCp type vector, and YIp5 (K. Struhl et al., Proc. Natl. Acad. Sci. USA, 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F: Tuite et aL, EMBO J., 1, 603 (1982), and are readily available by known methods.

Since an auxotrophy marker cannot be used as a selective marker upon transformation for a brewery yeast, for example, a geneticin-resistant gene (G418r), a copper-resistant gene (CUP1) (Marin et al., Proc. Natl. Acad. Sci. USA, 81, 337 1984) or a cerulenin-resistant gene (fas2m, PDR4) (Junji mokoshi et al., Biochemistry, 64, 660, 1992; and Hussain et al., Gene, 101: 149, 1991, respectively) may be used.

A vector constructed as described above is introduced into a host yeast. Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (Meth. Enzym., 194: 182 (1990)), a spheroplast method (Proc. Natl̂. Acad. Sci. USA, 75: 1929(1978)), a lithium acetate method (J. Bacteriology, 153: 163 (1983)), and methods described in Proc. Natl. Acad. Sci. USA, 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant.

Other general cloning techniques may be found, for example, in MOLECULAR CLONING 3rd End, and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce a desired alcoholic beverage with enhanced aroma and flavor with an elevated content of ester. In addition, yeasts to be selected by the yeast assessment method of the present invention described below can also be used. The target alcoholic beverages include, for example, but not limited to beer, beer-taste beverages such as sparkling liquor (*happoushu*), wine, whisky, sake and the like. Further, according to the present invention, desired alcoholic beverages with reduced ester level can be produced using brewery yeast in which the expression of the target gene was suppressed, if needed. That is to say, desired kind of alcoholic beverages with controlled (elevated or reduced) level of ester can be produced by controlling (elevating or reducing) production amount of ester using yeasts into which the vector of the present invention was introduced described above, yeasts in which expression of the polynucleotide (DNA) of the present invention described above was suppressed or yeasts selected by the yeast assessment method of the invention described below for fermentation to produce alcoholic beverages.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with an controlled content of ester. Thus, according to the present invention, alcoholic beverages with excellent aroma and flavor can be produced using the existing facility without increasing the cost.

### 5. Yeast assessment method of the invention

The present invention relates to a method for assessing a test yeast for its ester-producing capability by using a primer or a probe designed based on a nucleotide sequence of a esterase gene having the nucleotide sequence of SEQ ID NO:1. General techniques for such assessment method is known and is described in, for example, WO01/040514, Japanese Laid-Open Patent Application No. 8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the esterase gene, the existence of the gene or a sequence specific to the gene is determined in the test yeast genome obtained. The primer or the probe may be designed according to a known technique.

Detection of the gene or the specific sequence may be carried out by employing a known technique. For example, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of the yeast to produce ester as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present invention, a test yeast is cultured to measure an expression level of the esterase gene having the nucleotide sequence of SEQ ID NO: 1 to assess the test yeast for its ester-producing capability. In measuring an expression level of the esterase gene, the test yeast is cultured and then mRNA or a protein resulting from the esterase gene is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the esterase gene having the nucleotide sequence of SEQ ID NO: 1 are measured to select a test yeast with the gene expression level according to the target capability of producing ester, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the esterase gene having the nucleotide sequence of SEQ ID NO: 1 is measured in each yeast. By selecting a test yeast with the gene expressed higher or lower than that in the reference yeast, a yeast suitable for brewing alcoholic beverages can be selected.

Alternatively, test yeasts are cultured and a yeast with a higher or lower ester-producing capability or with a higher or lower esterase activity is selected, thereby selecting a yeast suitable for brewing desired alcoholic beverages.

In these cases, the test yeasts or the reference yeast may be, for example, a yeast introduced with the vector of the invention, a yeast in which an expression of a polynucleotide (DNA) of the invention has been controlled, an artificially mutated yeast or a naturally mutated yeast. The ester-producing capability can be measured by, for example, a method described in Method of J. Am. Soc. Brew. Chem. 49:152-157, 1991. Esterase activity can be measured by, for example, a method described in Appl. Microbiol. Biotechnol. 53: 596-600, 2000. The mutation treatment may employ any Methods including, for examples, physical methods such as ultraviolet irradiation and radiation irradiation, and chemical methods associated with treatments with drugs such as EMS (ethylmethane sulphonate) and N-methyl-N-nitrosoguanidine (*see, e.g.,* Yasuji Oshima Ed, BIOCHEMISTRY EXPERIMENTS vol. 39, Yeast Molecular Genetic Experiments, pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus, S. cerevisiae*, and *S. carlsbergensis*). According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used. Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used The reference yeast and the test yeasts may be selected from the above yeasts in any combination.

### EXAMPLES

Hereinafter, the present invention will be described in more detail with reference to working examples. The present invention, however, is not limited to the examples described below.

### Example 1: Cloning of Esterase Gene (nonScIAH1)

A specific novel esterase gene (nonScIAH1) (SEQ ID NO: 1) from a lager brewing yeast were found, as a result of a search utilizing the comparison database described in Japanese Patent Application Laid-Open No. 2004-283169. Based on the acquired nucleotide sequence information, primers nonScIAH1_for (SEQ ID NO: 3) and nonScIAH1_rv (SEQ ID NO: 4) were designed to amplify the full-length genes, respectively. PCR was carried out using chromosomal DNA of a genome sequencing strain, *Saccharomyces pastorianus* Weihenstephan 34/70 strain, also abbreviated to "W34/70 strain", as a template to obtain DNA fragments (about 0.7 kb) including the full-length gene of nonScIAH1.

The thus-obtained nonScIAH1 gene fragment was inserted into pCR2.1-TOPO vector (Invitrogen) by TA cloning. The nucleotide sequences of nonScIAH1 gene were analyzed according to Sanger's method (F. Sanger, Science, 214: 1215, 1981) to confirm the nucleotide sequence.

### Example 2: Analysis of Expression of nonScIAH1 Gene during Beer Fermentation Test

A beer fermentation test was conducted using a lager brewing yeast, *Saccharomyces pastorianus* W34/70 strain and then mRNA extracted from yeast cells during fermentation was analyzed by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

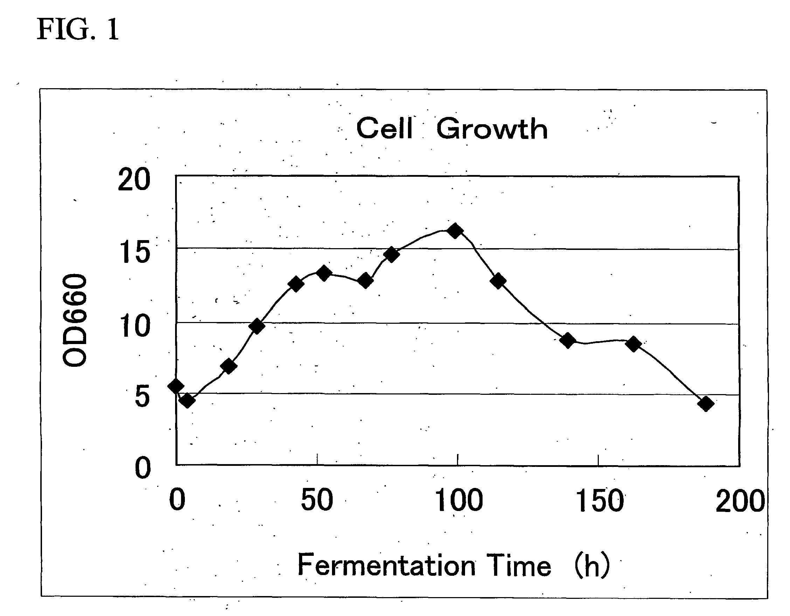

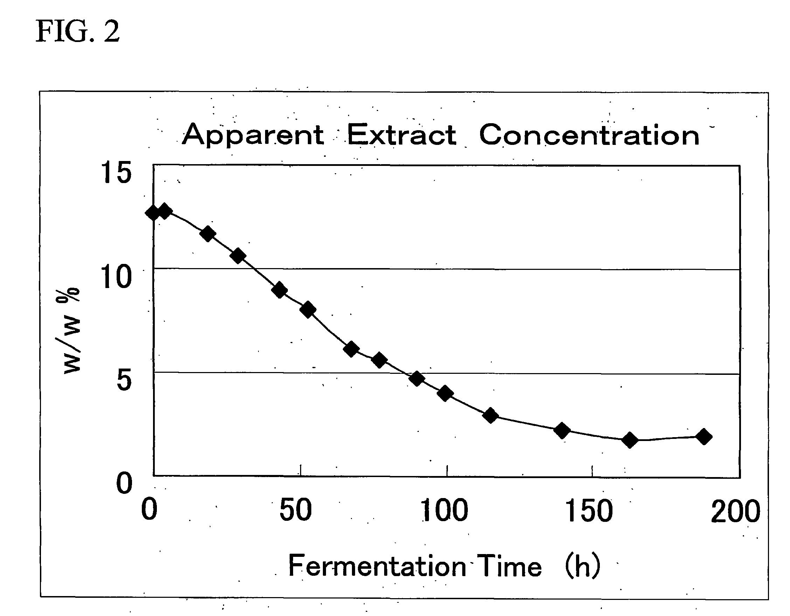

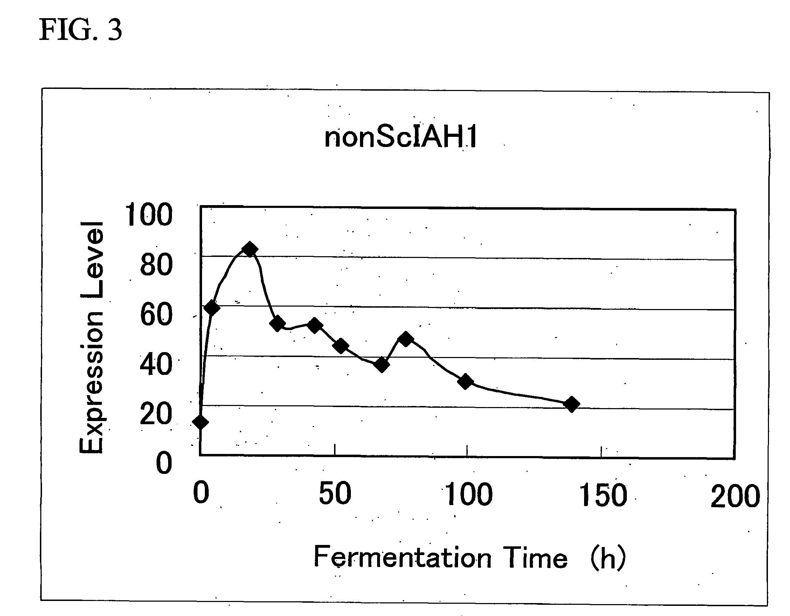

Sampling of fermentation liquor was performed with time, and variation with time of yeast growth amount (Fig. 1) and apparent extract concentration (Fig. 2) was observed. Simultaneously, sampling of yeast cells was performed, and the prepared mRNA was subjected to be biotin-labeled and was hybridized to a beer yeast DNA microarray. The signal was detected using GCOS; GeneChip Operating Software 1.0 (manufactured by Affymetrix Co.). Expression pattern of nonScIAH1 gene is shown in Figure 3. As a result, it was confirmed that nonScIAH1 gene was expressed in the general beer fermentation.

### Example 3: Construction of nonScIAH1 Gene Highly Expressed Strain

The nonScIAH1/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the nonScIAH1 high expression vector nonScIAH1/pYCGPYNot. pYCGPYNot is the YCp-type yeast expression vector. The inserted gene is highly expressed by the pyruvate kinase gene PYK1 promoter. The geneticin-resistant gene G418^{r} is included as the selection marker in the yeast, and the ampicillin-resistant gene Amp^{r} is included as the selection marker in *Escherichia coli.*

Using the high expression vector prepared by the above method, the strain *Saccharomyces pasteurianus* Weihenstephaner 34/70 was transformed by the method described in Japanese Patent Application Laid-open No. H7-303475. The transformant was selected in a YPD plate culture (1% yeast extract, 2% polypeptone, 2% glucose, 2% agar) containing 300 mg/L of geneticin.

### Example 4: Analysis of Amounts of Ester Formed in Beer Fermentation Test

A fermentation test was conducted under the following conditions using the parent strain (W34/70 strain) and the nonScIAH1 highly expressed strain obtained in Example 3.
Wort extract concentration: 12%
Wort volume: 2 L
Wort dissolved oxygen concentration: 8 ppm
Fermentation temperature: 15°C
Yeast pitching rate: 5 g/L

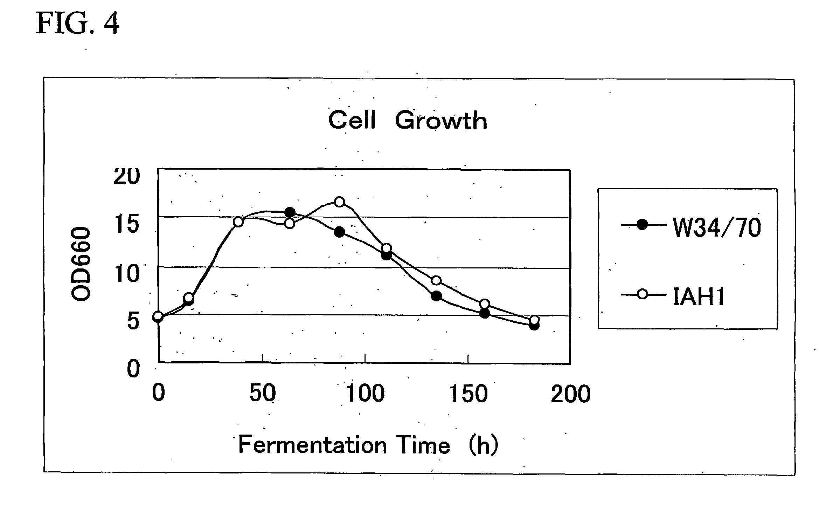

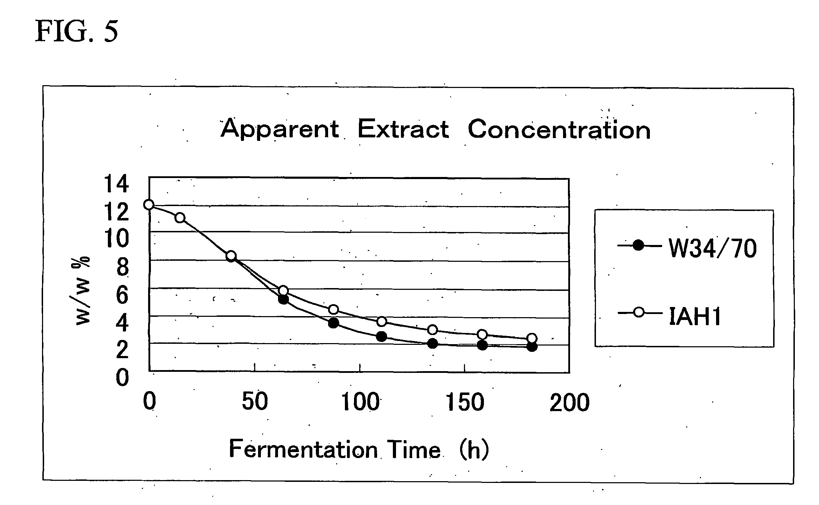

The fermentation broth was sampled over time to investigate the time-based changes in yeast growth (OD660) (Fig. 4) and extract consumption (Fig. 5). Quantification of higher alcohol and extract concentrations at completion of fermentation was carried out using head space gas chromatography (J. Am. Soc. Brew. Chem. 49:152-157,1991).

The amount of ethyl acetate formed at completion of fermentation was 24.0 ppm for the nonScIAH1 highly expressed strain in contrast to 34.4 ppm for the parent strain as described in Table 1. The amount of isoamyl acetate formed was 0.2 ppm for the nonScIAH1 highly expressed strain in contrast to 2.1 ppm for the parent strain. On the basis of these results, the amounts of ethyl acetate and isoamyl acetate formed were clearly demonstrated to decreased by 30 to 90% by high expression of nonScIAH1.

**Table 1**

| | Parent Strain (W34/70) | nonScIAH1 Highly Expressed Strain | |
|---|---|---|---|
| Ethyl acetate | 34.4 | 24.0 | (70%) |
| Isoamyl acetate | 2.1 | 0.2 | (9.5%) |

| | | | |
|---|---|---|---|
| Unit: ppm Values in parentheses indicate relative values versus the parent strain. | | | |

### Example 5: Disruption of nonScIAH1 Gene

Fragments for gene disruption were prepared by PCR using plasmids containing a drug resistance marker (pFA6a(G418r), pAG25(nat1), pAG32(hph)) as templates in accordance with a method described in the literature (Goldstein et al., Yeast, 15, 1541 (1999)). Primers consisting of nonScIAH1_delta_for (SEQ ID NO. 5) and nonScIAH1_delta_rv (SEQ ID NO. 6) were used for the PCR primers.

A spore clone (W34/70-2) isolated from brewer's yeast Saccharomyces pastorianus strain W34/70 was transformed with the fragments for gene disruption prepared as describe above.

Transformation was carried out according to the method described in Japanese Patent Application Laid-open No. H07-303475, and transformants were selected on YPD plate medium (1% yeast extract, 2% polypeptone, 2% glucose, 2% agar) containing geneticin at 300 mg/L, nourseothricin at 50 mg/L or hygromycin B at 200 mg/L.

### Example 6: Analysis of Amounts of Ester Formed in Beer Fermentation Test

A fermentation test was conducted under the following conditions using the parent strain (W34/70-2 strain) and the nonScIAH1 disrupted strain obtained in Example 5.
Wort extract concentration: 13%
Wort volume: 1 L
Wort dissolved oxygen concentration: 8 ppm
Fermentation temperature: 15°C
Yeast pitching rate: 5 g/L

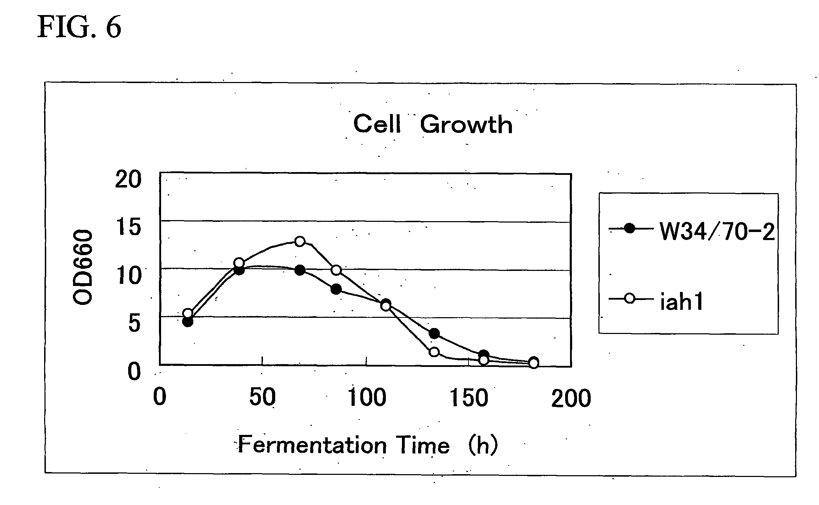

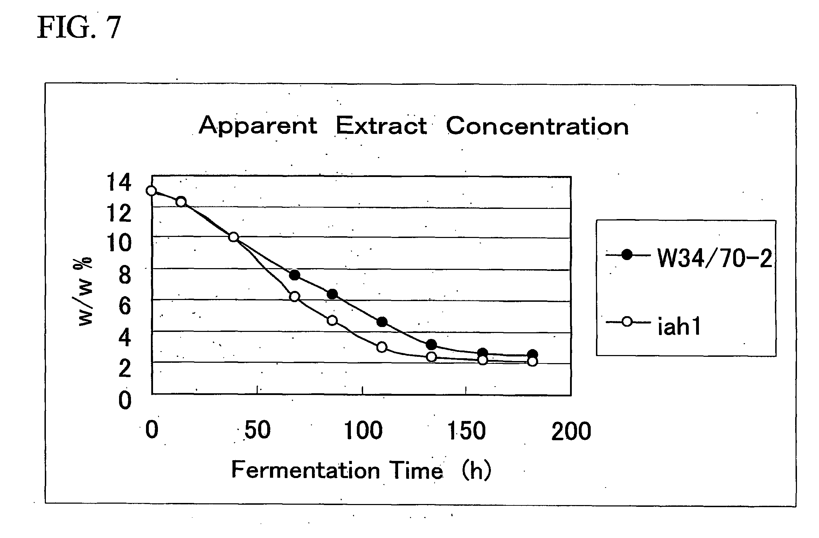

The fermentation broth was sampled over time to investigate the time-based changes in yeast growth (OD660) (Fig. 6) and extract consumption (Fig. 7). Quantification of ester concentration at completion of fermentation was carried out using head space gas chromatography (J. Am. Soc. Brew. Chem. 49:152-157, 1991).

The amount of ethyl acetate formed at completion of fermentation was 28.1 ppm for the nonScIAH1 disrupted strain in contrast to 26.2 ppm for the parent strain as described in Table 2.
The amount of isoamyl acetate formed was 2.9 ppm for the nonScIAH1 disrupted strain in contrast to 2.3 ppm for the parent strain, On the basis of these results, the amounts of ethyl acetate and isoamyl acetate formed were clearly demonstrated to increased by 7 to 26% by disruption of nonScIAH1.

**Table 2**

| | Parent Strain (W34/70-2) | nonScIAH1 disrupted Strain | |
|---|---|---|---|
| Ethyl acetate | 26.2 | 28.1 | (107%) |
| Isoamyl acetate | 2.3 | 2.9 | (126%) |

| | | | |
|---|---|---|---|
| Unit: ppm Values in parentheses indicate relative values versus the parent strain. | | | |

### INDUSTRIAL APPLICABILITY

According to the alcoholic beverage production method of the present invention, alcoholic beverages having superior aroma and flavor can be produced because the method can control the content of esters.

More specifically, according to the alcoholic beverage production method of the present invention, alcoholic beverages having superior aroma and flavor can be produced because the method can increase the content of esters which impart a florid aroma to products. In addition, in the case of malt beverages such as beer, for which an excessively high ester content is not preferred, alcoholic beverages having a more desirable aroma and flavor can be produced because the method can also decrease the amount of ester contained therein.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 40 amino acids thereof being deleted, substituted, inserted and/or added, and having a esterase activity; and
(d) a polynucleotide comprising a polynucleotide encoding a protein having an amino acid sequence having 80% or higher identity with the amino acid sequence of SEQ ID NO:2, and having a esterase activity.

2. The polynucleotide of Claim 1 selected from the group consisting of:
(g) a polynucleotide encoding a protein consisting of the amino acid sequence of SEQ ID NO: 2, or encoding an amino acid sequence of SEQ ID NO: 2 wherein 1 to 10 amino acids thereof is deleted, substituted, inserted, and/or added, and wherein said protein has a esterase activity; and
(h) a polynucleotide encoding a protein having 90% or higher identity with the amino acid sequence of SEQ ID NO: 2, and having a esterase activity.

3. The polynucleotide of Claim 1 comprising a polynucleotide consisting of SEQ ID NO: 1.

4. The polynucleotide of Claim 1 comprising a polynucleotide encoding a protein consisting of SEQ ID NO: 2.

5. The polynucleotide of any one of Claims 1 to 4, wherein the polynucleotide is DNA.

6. A polynucleotide selected from the group consisting of:
(j) a polynucleotide encoding RNA of a nucleotide sequence complementary to a transcript of the polynucleotide (DNA) according to Claim 5;
(k) a polynucleotide encoding RNA that represses the expression of the polynucleotide (DNA) according to Claim 5 through RNAi effect;
(I) a polynucleotide encoding RNA having an activity of specifically cleaving a transcript of the polynucleotide (DNA) according to Claim 5; and
(m) a polynucleotide encoding RNA that represses expression of the polynucleotide (DNA) according to Claim 5 through co-suppression effect.

7. A protein encoded by the polynucleotide of any one of Claims 1 to 5.

8. A vector comprising the polynucleotide of any one of Claims 1 to 5.

9. A vector comprising the polynucleotide of Claim 6.

10. A yeast comprising the vector of Claim 8 or 9.

11. The yeast of Claim 10, wherein a ester-producing ability is decreased by introducing the vector of Claim 8.

12. A yeast, wherein an expression of the polynucleotide (DNA) of Claim 5 is repressed by introducing the vector of Claim 9, or by disrupting a gene related to the polynucleotide (DNA) of Claim 5.

13. The yeast of Claim 11, wherein a ester-producing ability is decreased by increasing an expression level of the protein of Claim 7.

14. A method for producing an alcoholic beverage by using the yeast of any one of Claims 10 to 13.

15. The method for producing an alcoholic beverage of Claim 14, wherein the brewed alcoholic beverage is a malt beverage.

16. The method for producing an alcoholic beverage of Claim 14, wherein the brewed alcoholic beverage is wine.

17. A method for assessing a test yeast for its ester-producing capability, comprising using a primer or a probe designed based on a nucleotide sequence of a esterase gene having the nucleotide sequence of SEQ ID NO: 1.

18. A method for assessing a test yeast for its ester-producing capability, comprising:
culturing a test yeast; and measuring an expression level of a esterase gene having the nucleotide sequence of SEQ ID NO: 1.

19. A method for selecting a yeast, comprising: culturing test yeasts; quantifying the protein according to Claim 7 or measuring an expression level of a esterase gene having the nucleotide sequence of SEQ ID NO: 1; and selecting a test yeast having said protein amount or said gene expression level according to a target capability of producing ester.

20. The method for selecting a yeast according to Claim 19, comprising: culturing a reference yeast and test yeasts; measuring an expression level of a esterase gene having the nucleotide sequence of SEQ ID NO: 1 in each yeast; and selecting a test yeast having the gene expressed higher or lower than that in the reference yeast.

21. The method for selecting a yeast according to Claim 19, comprising: culturing a reference yeast and test yeasts; quantifying the protein according to Claim 7 in each yeast; and selecting a test yeast having said protein for a larger or smaller amount than that in the reference yeast.

22. A method for producing an alcoholic beverage comprising: conducting fermentation for producing an alcoholic beverage using the yeast according to any one of Claims 10 to 13 or a yeast selected by the method according to any one of Claims 19 to 21; and adjusting the production amount of ester.

## Patentansprüche

1. Polynucleotid, das ausgewählt ist aus der Gruppe bestehend aus:
(a) einem Polynucleotid umfassend ein Polynucleotid, welches aus der Nucleotidsequenz der SEQ ID NO:1 besteht;
(b) einem Polynucleotid umfassend ein Polynucleotid, das ein Protein codiert, welches aus der Aminosäuresequenz der SEQ ID NO:2 besteht;
(c) einem Polynucleotid umfassend ein Polynucleotid, das ein Protein codiert, welches aus der Aminosäuresequenz der SEQ ID NO: 2 besteht, in welcher eine bis 40 Aminosäuren deletiert, substituiert, inseriert und/oder hinzugefügt wurden, und eine Esterase-Aktivität hat; und
(d) einem Polynucleotid umfassend ein Polynucleotid, das ein Protein codiert, welches eine Aminosäuresequenz mit 80%iger oder höherer Identität zu der Aminosäuresequenz der SEQ ID NO: 2 hat und eine Esterase-Aktivität hat.

2. Polynucleotid nach Anspruch 1, das ausgewählt ist aus der Gruppe bestehend aus:
(g) einem Polynucleotid, das ein Protein codiert, welches aus der Aminosäuresequenz der SEQ ID NO:2 besteht, oder eine Aminosäuresequenz der SEQ ID NO:2 codiert, in welcher 1 bis 10 Aminosäuren deletiert, substituiert, inseriert und/oder hinzugefügt sind, und wobei das Protein eine Esterase-Aktivität hat; und
(h) einem Polynucleotid, das ein Protein codiert, welches eine 90%ige oder höhere Identität zu der Aminosäuresequenz der SEQ ID NO: 2 hat und eine Esterase-Aktivität hat.

3. Polynucleotid nach Anspruch 1, umfassend ein Polynucleotid, welches aus der SEQ ID NO:1 besteht.

4. Polynucleotid nach Anspruch 1, umfassend ein Polynucleotid, welches ein Protein codiert, das aus der SEQ ID NO: 2 besteht.

5. Polynucleotid nach einem der Ansprüche 1 bis 4, wobei das Polynucleotid DNA ist.

6. Polynucleotid, das ausgewählt ist aus der Gruppe bestehend aus:
(j) einem Polynucleotid, das eine RNA einer Nucleotidsequenz codiert, welche komplementär zu einem Transkript des Polynucleotids (DNA) gemäß Anspruch 5 ist;
(k) einem Polynucleotid, das eine RNA codiert, welche die Expression des Polynucleotids (DNA) gemäß Anspruch 5 durch einen RNAi-Effekt reprimiert;
(l) einem Polynucleotid, das eine RNA codiert, welche eine Aktivität zur spezifischen Spaltung eines Transkripts des Polynucleotids (DNA) gemäß Anspruch 5 hat; und
(m) einem Polynucleotid, das eine RNA codiert, welche die Expression des Polynucleotids (DNA) gemäß Anspruch 5 durch einen Co-Suppressionseffekt reprimiert.

7. Protein, welches durch das Polynucleotid nach einem der Ansprüche 1 bis 5 codiert wird.

8. Vektor, welcher das Polynucleotid nach einem der Ansprüche 1 bis 5 umfasst.

9. Vektor, welcher das Polynucleotid nach Anspruch 6 umfasst.

10. Hefe, welche den Vektor nach Anspruch 8 oder 9 umfasst.

11. Hefe nach Anspruch 10, in der eine Ester-produzierende Fähigkeit durch das Einführen des Vektors nach Anspruch 8 verringert ist.

12. Hefe, in der eine Expression des Polynucleotids (DNA) nach Anspruch 5 durch das Einführen des Vektors nach Anspruch 9 oder durch Zerstören eines Gens, welches sich auf das Polynucleotid nach Anspruch 5 bezieht, reprimiert ist.

13. Hefe nach Anspruch 11, in der eine Ester-produzierende Fähigkeit durch die Zunahme eines Expressionsspiegels des Proteins nach Anspruch 7 verringert ist.

14. Verfahren zur Herstellung eines alkoholischen Getränks durch Verwendung der Hefe nach einem der Ansprüche 10 bis 13.

15. Verfahren zur Herstellung eines alkoholischen Getränks nach Anspruch 14, wobei das gebraute alkoholische Getränk ein Malzgetränk ist.

16. Verfahren zur Herstellung eines alkoholischen Getränks nach Anspruch 14, wobei das gebraute alkoholische Getränk Wein ist.

17. Verfahren zur Beurteilung einer Test-Hefe bezüglich ihrer Ester-produzierenden Fähigkeit, umfassend die Verwendung eines Primers oder einer Sonde, welche(r) basierend auf einer Nucleotidsequenz eines Esterase-Gens, welches die Nucleotidsequenz der SEQ ID NO:1 hat, entworfen wurde.

18. Verfahren zur Beurteilung einer Test-Hefe bezüglich ihrer Ester-produzierenden Fähigkeit, umfassend: Züchten einer Test-Hefe; und Messen eines Expressionsspiegels eines Esterase-Gens, welches die Nucleotidsequenenz der SEQ ID NO: 1 hat.

19. Verfahren zur Auswahl einer Hefe, umfassend: Züchten von Test-Hefen; Quantifizieren des Proteins gemäß Anspruch 7 oder Messen eines Expressionsspiegels eines Esterase-Gens, welches die Nucleotidsequenz der SEQ ID NO: 1 hat; und Auswahl einer Test-Hefe, welche den Proteingehalt oder den Expressionsspiegel gemäß einer Zielfähigkeit zur Esterproduktion hat.

20. Verfahren zur Auswahl einer Hefe gemäß Anspruch 19, umfassend: Züchten einer Referenz-Hefe und von Test-Hefen; Messen eines Expressionsspiegels eines Esterase-Gens, welches die Nucleotidsequenz der SEQ ID NO: 1 hat, in jeder Hefe; und Auswahl einer Test-Hefe, in welcher das Gen höher oder niedriger als das in der Referenz-Hefe exprimiert ist.

21. Verfahren zur Auswahl einer Hefe gemäß Anspruch 19, umfassend: Züchten einer Referenz-Hefe und von Test-Hefen; Quantifizieren des Proteins gemäß Anspruch 7 in jeder Hefe; und Auswahl einer Test-Hefe, welche das Protein in einer größeren oder kleineren Menge als das in der Referenz-Hefe hat.

22. Verfahren zur Herstellung eines alkoholischen Getränks umfassend: Ausführen einer Fermentation zur Produktion eines alkoholischen Getränks unter Verwendung einer Hefe gemäß einem der Ansprüche 10 bis 13 oder einer Hefe, welche durch das Verfahren gemäß einem der Ansprüche 19 bis 21 ausgewählt ist; und Anpassen der Produktionsmenge des Esters.

## Revendications

1. Polynucléotide sélectionné dans le groupe consistant en :
(a) un polynucléotide comprenant un polynucléotide consistant en la séquence nucléotidique de SEQ ID NO : 1 ;
(b) un polynucléotide comprenant un polynucléotide codant pour une protéine consistant en la séquence en acides aminés de SEQ ID NO : 2 ;
(c) un polynucléotide comprenant un polynucléotide codant pour une protéine consistant en la séquence en acides aminés de SEQ ID NO : 2 avec un à 40 acides aminés de celle-ci étant délétés, substitués, insérés et/ou ajoutés, et possédant une activité estérase ; et
(d) un polynucléotide comprenant un polynucléotide codant pour une protéine ayant une séquence en acides aminés présentant 80 % ou plus d'identité avec la séquence en acides aminés de SEQ ID NO : 2 et possédant une activité estérase.

2. Polynucléotide selon la revendication 1, choisi dans le groupe consistant en :
(g) un polynucléotide codant pour une protéine consistant en la séquence en acides aminés de SEQ ID NO : 2, ou codant pour une séquence en acides aminés de SEQ ID NO : 2 dans laquelle 1 à 10 acides aminés de celle-ci sont délétés, substitués, insérés et/ou ajoutés, et où ladite protéine possède une activité estérase ; et
(h) un polynucléotide codant pour une protéine présentant 90 % ou plus d'identité avec la séquence en acides aminés de SEQ ID NO : 2 et possédant une activité estérase.

3. Polynucléotide selon la revendication 1, comprenant un polynucléotide consistant en SEQ ID NO : 1.

4. Polynucléotide selon la revendication 1, comprenant un polynucléotide codant pour une protéine consistant en SEQ ID NO : 2.

5. Polynucléotide selon l'une quelconque des revendications 1 à 4, où le polynucléotide est de l'ADN.

6. Polynucléotide sélectionné dans le groupe consistant en :
(j) un polynucléotide codant pour un ARN d'une séquence nucléotidique complémentaire d'un transcrit du polynucléotide (ADN) selon la revendication 5 ;
(k) un polynucléotide codant pour un ARN qui réprime l'expression du polynucléotide (ADN) selon la revendication 5 par effet d'ARNi ;
(l) un polynucléotide codant pour un ARN possédant une activité de clivage spécifique d'un transcrit du polynucléotide (ADN) selon la revendication 5 ; et
(m) un polynucléotide codant pour un ARN qui réprime l'expression du polynucléotide (ADN) selon la revendication 5 par de co-suppression.

7. Protéine codée par le polynucléotide selon l'une quelconque des revendications 1 à 5.

8. Vecteur comprenant le polynucléotide selon l'une quelconque des revendications 1 à 5.

9. Vecteur comprenant le polynucléotide selon la revendication 6.

10. Levure comprenant le vecteur selon la revendication 8 ou 9.

11. Levure selon la revendication 10, dans laquelle une capacité de produire un ester est diminuée en introduisant le vecteur selon la revendication 8.

12. Levure, dans laquelle une expression du polynucléotide (ADN) selon la revendication 5 est réprimée en introduisant le vecteur selon la revendication 9, ou en perturbant un gène associé au polynucléotide (ADN) selon la revendication 5.

13. Levure selon la revendication 11, dans laquelle une capacité de produire un ester est diminuée en augmentant un taux d'expression de la protéine selon la revendication 7.

14. Procédé de production d'une boisson alcoolisée en utilisant la levure selon l'une quelconque des revendications 10 à 13.

15. Procédé de production d'une boisson alcoolisée selon la revendication 14, dans lequel la boisson alcoolisée brassée est une boisson à base de malt.

16. Procédé de production d'une boisson alcoolisée selon la revendication 14, dans lequel la boisson alcoolisée brassée est du vin.

17. Procédé d'évaluation d'une levure à tester pour sa capacité de produire un ester, comprenant l'utilisation d'une amorce ou d'une sonde conçue d'après une séquence nucléotidique d'un gène d'estérase ayant la séquence nucléotidique de SEQ ID NO : 1.

18. Procédé d'évaluation d'une levure à tester pour sa capacité de produire un ester, comprenant : la culture d'une levure à tester ; et la mesure d'un taux d'expression d'un gène d'estérase ayant la séquence nucléotidique de SEQ ID NO : 1.

19. Procédé de sélection d'une levure, comprenant : la culture de levures à tester ; la quantification de la protéine selon la revendication 7 ou la mesure d'un taux d'expression d'un gène d'estérase ayant la séquence nucléotidique de SEQ ID NO : 1 ; et la sélection d'une levure à tester ayant ladite quantité de protéine ou ledit taux d'expression de gène selon une capacité cible de produire un ester.

20. Procédé de sélection d'une levure selon la revendication 19, comprenant : la culture d'une levure de référence et des levures à tester ; la mesure d'un taux d'expression d'un gène d'estérase ayant la séquence nucléotidique de SEQ ID NO : 1 dans chaque levure ; et la sélection d'une levure à tester ayant le gène exprimé supérieur ou inférieur à celui dans la levure de référence.

21. Procédé de sélection d'une levure selon la revendication 19, comprenant : la culture d'une levure de référence et des levures à tester ; la quantification de la protéine selon la revendication 7 dans chaque levure ; et la sélection d'une levure à tester ayant ladite protéine pour une quantité supérieure ou inférieure à celle dans la levure de référence.

22. Procédé de production d'une boisson alcoolisée comprenant : la conduite d'une fermentation pour produire une boisson alcoolisée en utilisant la levure selon l'une quelconque des revendications 10 à 13 ou une levure sélectionnée par le procédé selon l'une quelconque des revendications 19 à 21 ; et l'ajustement de la quantité de production d'ester.


### SEQUENCE LISTING

<110> Suntory Limited
<120> Esterase gene and use thereof
<130> P1227 EP S3
<140> PCT/JP2006/318466
   <141> 2006-09-12
<150> JP2005-265918
   <151> 2005-09-13
<150> JP2006-047561
   <151> 2006-02-23
<160> 6
<210> 1
   <211> 717
   <212> DNA
   <213> Saccharomyces sp.
<400> 1
   atggaatacg agaagtttct attgttcggg gattccatca ctgaatttgc attcaatact     60
   aggcccacag aggatgataa agattattat gctcttggag ccgcgttggt taacgagtat    120
   acaagaaaaa tggacatcct tcagcgagga ttcaaagggt acaactccaa gtgggcactg    180
   aaggttcttc ctgaaatctt aaacaatgag cccaacattg ccatggccac catctttttc    240
   ggtgccaacg atgcatgctt agctggccct caatgtgtgc ctctcttaga atttgtcgat    300
   aatataggtc aaatggtgtc tgtcatgaaa gctcatcacg ttcttcctat tattgtaggc    360
   ccggcactgg tggatagaga gaagtgggaa aaagcaaggg ctgacgaaac agctctcgga    420
   tatgtgcgta ccaacgagaa ctttacagtc tattctgatg cgttagcaaa gctggccgac    480
   gacgaaaaaa tccccttcgt gaacttgaat aaagcgtttc aaaagataga cggtgatgcc    540
   tggaagagcc tactaacgga tggactacac ttctctgggg aaggatacaa aatcttccat    600
   gatgaattga tcaaagccat cgaggcacac tatcctcaat accatcctaa taacatggaa    660
   tacaaactaa cagactggag agatgtgttg gatgacggct ccaacatttt gtcttaa       717
<210> 2
   <211> 238
   <212> PRT
   <213> Saccharomyces sp.
<400> 2
   Met Glu Tyr Glu Lys Phe Leu Leu Phe Gly Asp Ser Ile Thr Glu Phe
   1               5                   10                  15
   Ala Phe Asn Thr Arg Pro Thr Glu Asp Asp Lys Asp Tyr Tyr Ala Leu
               20                  25                  30
   Gly Ala Ala Leu Val Asn Glu Tyr Thr Arg Lys Met Asp Ile Leu Gln
           35                  40                  45
   Arg Gly Phe Lys Gly Tyr Asn Ser Lys Trp Ala Leu Lys Val Leu Pro
       50                  55                  60
   Glu Ile Leu Asn Asn Glu Pro Asn Ile Ala Met Ala Thr Ile Phe Phe
   65                  70                  75                  80
   Gly Ala Asn Asp Ala Cys Leu Ala Gly Pro Gln Cys Val Pro Leu Leu
                   85                  90                  95
   Glu Phe Val Asp Asn Ile Gly Gln Met Val Ser Val Met Lys Ala His
               100                 105                 110
   His Val Leu Pro Ile Ile Val Gly Pro Ala Leu Val Asp Arg Glu Lys
           115                 120                 125
   Trp Glu Lys Ala Arg Ala Asp Glu Thr Ala Leu Gly Tyr Val Arg Thr
       130                 135                 140
   Asn Glu Asn Phe Thr Val Tyr Ser Asp Ala Leu Ala Lys Leu Ala Asp
   145                 150                 155                 160
   Asp Glu Lys Ile Pro Phe Val Asn Leu Asn Lys Ala Phe Gln Lys Ile
                   165                 170                 175
   Asp Gly Asp Ala Trp Lys Ser Leu Leu Thr Asp Gly Leu His Phe Ser
               180                 185                 190
   Gly Glu Gly Tyr Lys Ile Phe His Asp Glu Leu Ile Lys Ala Ile Glu
           195                 200                 205
   Ala His Tyr Pro Gln Tyr His Pro Asn Asn Met Glu Tyr Lys Leu Thr
       210                 215                 220
   Asp Trp Arg Asp Val Leu Asp Asp Gly Ser Asn Ile Leu Ser
   225                 230                 235
<210> 3
   <211> 40
   <212> DNA
   <213> Artificial Sequence
<220>
   <221> source
   <223> /note="Description of artificial sequence: Primer"
<400> 3
   gagctcatag cggccatgga atacgagaag tttctattgt 40
<210> 4
   <211> 42
   <212> DNA
   <213> Artificial Sequence
<220>
   <221> source
   <223> /note="Description of artificial sequence: Primer"
<400> 4
   ggatcctatg cggccgcctc attattgctt ttttccctga aa 42
<210> 5
   <211> 70
   <212> DNA
   <213> Artificial Sequence
<220>
   <221> source
   <223> /note="Description of artificial sequence: Primer"
<400> 5
   aggtaccttg ggcattatca attttttggc gcattacatt gtagtggaac ccttgacagt     60
   cttgacgtgc                                                           70
<210> 6
   <211> 70
   <212> DNA
   <213> Artificial Sequence
<220>
   <221> source
   <223> /note="Description of artificial sequence: Primer"
<400> 6
   gggcgttatg tgaagggaaa ataagtcaat acgtacaaaa ggactactgc cgcacttaac     60
   ttcgcatctg                                                           70